# EUROPEAN PATENT APPLICATION

(11) **EP 4 300 389 A1**
(43) Date of publication of application: **03.01.2024**
(21) Application number: 23164950.0
(22) Date of filing: 29.03.2023
(51) Int. Cl.: G06Q 10/063, G06F 7/00, G06Q 10/0633, G06Q 50/22, G16H 10/00, G16H 20/00, G16Z 99/00

(54) **METHOD FOR GENERATING PREDICTING MODEL, INFORMATION PROCESSING APPARATUS, AND PREDICTING MODEL GENERATING PROGRAM**

(30) Priority: 30.06.2022 JP 2022105955
(71) Applicant: FUJITSU LIMITED, Kawasaki-shi, Kanagawa 211-8588 (JP)
(72) Inventor: NAKAO, Manabu, Kawasaki-shi, Kanagawa, 211-8588 (JP); INOMATA, Akihiro, Kawasaki-shi, Kanagawa, 211-8588 (JP); SAWASAKI, Naoyuki, Kawasaki-shi, Kanagawa, 211-8588 (JP)
(74) Representative: Hoffmann Eitle

(57) **Abstract**

A computer-implemented method for generating a predicting model includes: accumulating a plurality of combinations of a parameter representing a branch condition and a branch probability in relation to one or more condition branch components in a workflow; and generating, using the plurality of combinations accumulated, a model that predicts a branch probability corresponding to a parameter used when a particular condition branch component among the one or more condition branch components is used.

## Description

### [Technical Field]

The embodiment discussed herein relates to a method for generating a predicting model, an information processing apparatus, and a predicting model generating program.

### [Background Technique]

Governments of countries and local municipalities may develop policies for health services to residents, for example.

For example, there is a technique that vectorizes health care data and estimates the effect of executing a policy on the basis of a result of training using the percentage of achievement of a target and a result of actual measurement of the policy effect information as response variables.

### [Related Art Reference]

### [Patent Document]

Japanese Laid-open Patent Publication No.2021-89523

### [Summary of Invention]

### [Problems to be Solved by Invention]

However, in the practice of policy development, policy plans are prepared on the basis of consideration, experience, and assumptions, which makes it difficult to achieve the policy index (Key Performance Indicator: KPI) goal.

As one aspect, the object of the present embodiment is to generate a highly accurate predicting model with low computational complexity.

### [Means to Solve the Problem]

According to an aspect of the embodiments, a computer-implemented method for generating a predicting model includes: accumulating a plurality of combinations of a parameter representing a branch condition and a branch probability in relation to one or more condition branch components in a workflow; and generating, using the plurality of combinations accumulated, a model that predicts a branch probability corresponding to a parameter used when a particular condition branch component among the one or more condition branch components is used.

### [Effect of Invention]

As one aspect, a highly accurate predicting model can be generated with low computational complexity.

### [Brief Description of Drawings]

FIG. 1 is a diagram illustrating a policy development according to a related example;
FIG. 2 is a diagram illustrating a predicting model according to one embodiment;
FIG. 3 is a diagram illustrating propagation of influence between components in a predicting model according to the one embodiment;
FIG. 4 is a diagram illustrating a service executing component included in the predicting model of the one embodiment;
FIG. 5 is a diagram illustrating calculation of an effect and a cost of an overall policy in the predicting model of the one embodiment;
FIG. 6 is a diagram illustrating cumulative information accumulated for training each component in the predicting model of the one embodiment;
FIG. 7 is a diagram illustrating an accumulating process of the cumulative information of the one embodiment;
FIG. 8 is a diagram illustrating an example of a generating process of a regression model from a condition branch component included in the predicting model of the one embodiment;
FIG. 9 is a diagram illustrating an example of a generating process of a regression model from a service executing component included in the predicting model of the one embodiment;
FIG. 10 is a diagram illustrating the cumulative information accumulated for training each component by a predicting model according to a first modification;
FIG. 11 is a diagram illustrating an accumulating process of the cumulative information of the first modification;
FIG. 12 is a flow diagram illustrating a constructing process of the predicting model of the one embodiment;
FIG. 13 is a flow diagram illustrating a calculating process of a probability using the predicting model of the one embodiment;
FIG. 14 is a flow diagram illustrating a first example of a constructing process of the predicting model of the first modification;
FIG. 15 is a flow diagram illustrating a first example of a calculating process of a probability using the predicting model of the first modification;
FIG. 16 is a flow diagram illustrating a second example of a constructing process of the predicting model of the first modification;
FIG. 17 is a diagram illustrating a second example of a calculating process of a probability using the predicting model of the first modification; and
FIG. 18 is a diagram schematically illustrating an example of a hardware configuration of an information processing apparatus of the one embodiment and the modifications.

### [Description of Embodiment(s)]

### (A) Related Example:

In order to prolong the healthy life expectancy of citizens, the national government is urging local municipality governments to develop data health plans and implement efficient health projects.

An example of health project is a policy that executes medical consultation and health guidance by specialists, such as a CKD (chronic kidney disease) follow-up policy for citizens who are judged to be at high risk of illness in health checkup.

On the basis of a numerical value of the medical examination value as a result of health checkup as a criterion for judgement, the policy recommends such a citizen to consult a medical specialist. For example, if the urinary protein is equal to or larger than 2+ or the eGFR 60 ml/min/ of 1.73 m², an examination by a specialist is recommended.

In this policy development, if the numerical value serving as the criterion of the judgement on a medical examination value is changed, the influence of the policy may change.

Although it is desired to loosen the judgement criterion to increase citizens that will consult specialists, excessively loosening the judgement criterion would excessively increase the consultation objects and there is a possibility that the specialists are unable to treat them all.

A target policy is expressed in a workflow consisting of multiple condition branches and multiple service executions, for example. On the basis of state data of an individual serving as an object of the policy, multiple condition branch are sequentially traced, and "which service is to be allocated" is determined.

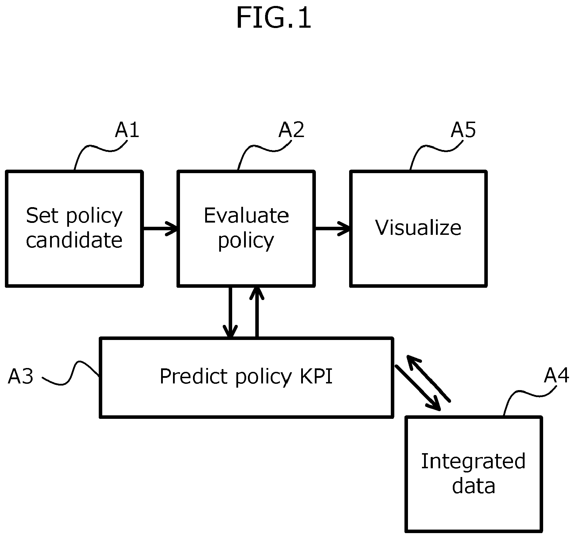

As an evaluation index of a policy, the effective, the cost, the people count to receive the service, and the like may be used. In a CKD follow-up policy, the effect is exemplified by an extent of reduction in onset people of chronic kidney disease (=number of citizens × onset ratio), the cost is exemplified by cost on the municipality to carry out health checkup and treatment by physicians, and the people count to receive the service is exemplified by the number of persons to receive services of health checkup and the number of people receiving Health checkup and services of physicians as the number of people receiving services. FIG. 1 is a diagram illustrating a policy development according to the related example.

In regard of a policy candidate, there is a technique that predicts a KPI of a policy from integrated data for individual including various types of data and supports decision-making in policy developing.

In FIG. 1, in setting a policy candidate represented by the reference sign A1, a policy target of "reducing the prevalence of obesity in adults in the target regional area to 25 %", a policy candidate of "implementing exercise and nutritional guidance by physicians for young people living in the regional area", and a KPI of "prevalence of obesity in adults in the regional area" are set.

In the reference sign A2, the set policy candidate is evaluated.

In the reference sign A3, a policy KPI is predicted. For example, the probability of becoming obese within three years when the policy candidate is executed is predicted (at three levels of high, medium, and low)" for individuals in the target regional area.

In the reference sign A4, the integrated data for predicting a policy KPI is referred to. The integrated data may include, for example, a history of daily health-information (such as the number of steps), a history of diagnosis results, a history of lifestyle habits, and a history of examination results for each individual local resident.

In the reference sign A5, the policy evaluation is visualized.

This calculates the probability of becoming obese for each individual and then calculates the prevalence of obesity within the area.

However, there is a possibility that the calculation time of the effect prediction when the policy is changed increases. Since the probability of becoming obese is calculated for each individual, a prediction using data of hundreds of thousands of citizens may take several to several dozen minutes and the result is not immediately output.

A policy developer sometimes wishes to examine useful policy change plans by experimenting with affects caused by modification to this part of a policy in a development stage of the policy. In addition, the developer sometimes wishes to shorten the calculation time to accelerate the cycles for examining the change.

### (B) Embodiment:

Hereinafter, one embodiment will now be described with reference to the accompanying drawings. However, the following embodiment is merely illustrative and there is no intention to exclude the application of various modifications and techniques not explicitly described in the embodiment. Namely, the present embodiment can be variously modified and implemented without departing from the scope thereof. Further, each of the drawings can include additional functions not illustrated therein to the elements illustrated in the drawing. Hereinafter, like reference numbers in all the drawings designate the same functions, and therefore repetitious description is sometimes omitted.

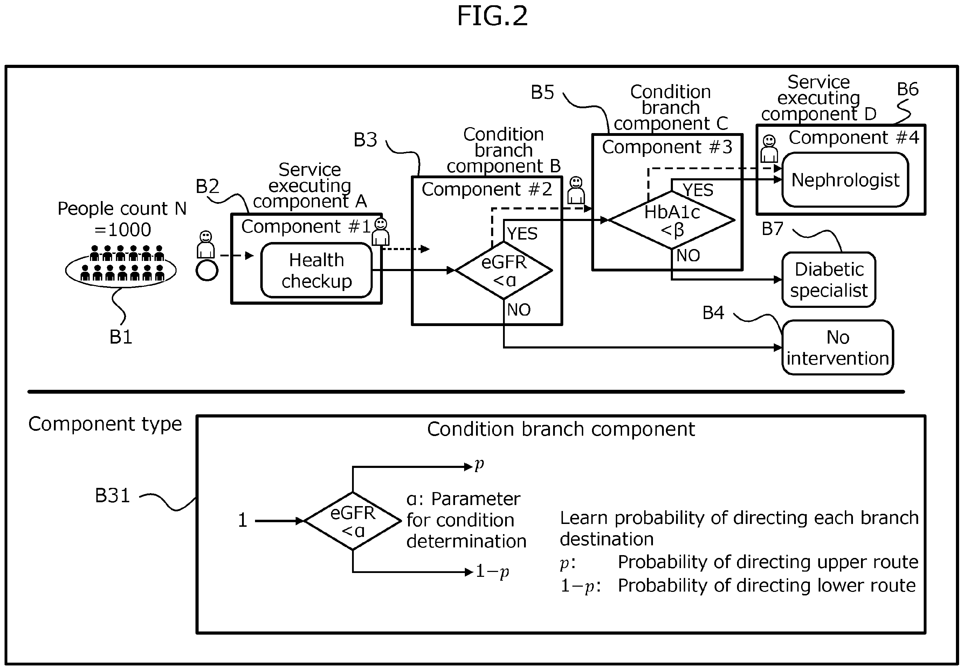

FIG. 2 is a diagram illustrating a predicting model according to the one embodiment.

A policy is modeled in the form of a workflow composed of a combination of condition branches and components such as service execution. Then, a people count that is to receive each service is predicted, using a model trained by accumulating information of a people flow and parameter from the actual values when each condition branch component was used in the past.

In the example of FIG. 2, people count N = 1000 is inputted into the reference sign B1.

In the reference sign B2, the component #1 as service executing component A is set to "health checkup".

In the reference sign B3, the component #2 as a condition branch component B is set to "eGFR<α".

As indicated in the reference sign B31, in a condition branch component, the probabilities of directing each branch destination is learned. When α is set to a parameter for condition determination, a probability of directing the upper route of the condition branch is p and a probability of directing the lower route of the condition branch is 1-p.

If "eGFR<α" is not satisfied (see NO route of the reference sign B3), it is determined that "no intervention" of a specialist is required for the citizen, as illustrated by the reference sign B4.

On the other hand, if "eGFR<α" is satisfied (see Yes route of the reference sign B3), as illustrated in the reference sign B5, the component #3 as the condition branch component C is set to "HbA1c<β".

If "HbA1c<β" is satisfied (see YES route of the reference sign B5), as illustrated in the reference sign B6, the component #4 as the condition branch component D is set to "nephrologist", which means that intervention of a "nephrologist" is required for the citizen.

On the other hand, if "HbA1c<β" is not satisfied (see NO route of the reference sign B5), it is determined that intervention of a "diabetic specialist" is required for the citizen, as illustrated in the reference sign B7.

In the example illustrated in FIG. 2, as indicated by the dotted arrows, the people counts flowing sequentially into the component #1, the component #2, the component #3, and the component #4 are predicted.

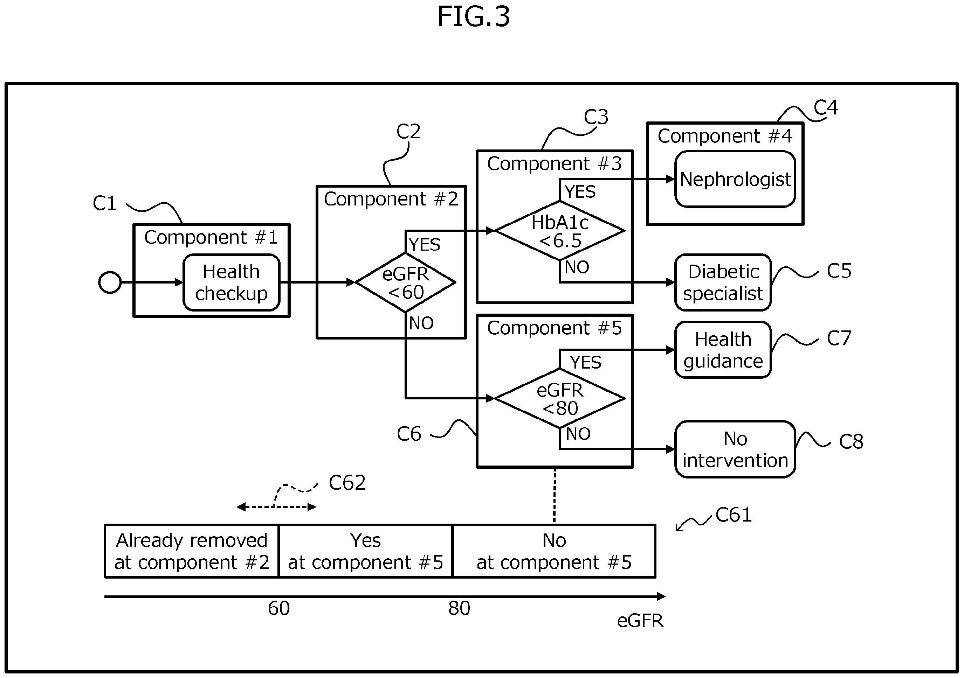

FIG. 3 is a diagram illustrating propagation of influence between components in a predicting model according to the one embodiment.

A downstream component may be affected by an upstream component. A downstream component is affected by a component used at the upstream side and a parameter value of each component.

In the reference sign C1, "health checkup" is set as the component #1.

In the reference sign C2, "eGFR<60" is set as the component #2.

If "eGFR<60" is satisfied (see Yes route of the reference sign C2), "HbA1c<6.5" is set as the component #3 in the reference sign C3.

If the "HbA1c<6.5" is satisfied (see Yes route of the reference sign C3), "nephrologist" is set as the component #4 in the reference sign C4, and it is determined that intervention of a "nephrologist" is required for the citizen.

On the other hand, if the "HbA1c<6.5" is not satisfied (see NO route of the reference sign C3), it is determined in the reference sign C5 that intervention of a "diabetic specialist" is required for the citizen.

If "eGFR<60" is not satisfied in the reference sign C2 (see NO route of the reference sign C2), "eGFR<80" is set as the component #5 in the reference sign C6.

If "eGFR<80" is satisfied (see Yes route of the reference sign C6), it is determined that "health guidance" to the citizen is required in the reference sign C7.

On the other hand, if the "eGFR<80" is not satisfied (see NO route of the reference sign C6), it is determined in the reference sign C8 that "No intervention" is required for the citizen.

As illustrated in the reference sign C61, the ratio of people flowing into the Yes route of the component #5 and people flowing into the NO route of the component #5 is affected by the distribution of people in the component #2.

As illustrated in the reference sign C62, if the determination threshold (60) of the parameter of the component #2 is changed, the range of people directed to the Yes route of the component #5 fluctuates.

Therefore, a people count that is to receive each service may be predicted, using a model trained by accumulating a combination of components as using the policy in addition to information of a people flow and a parameter from the actual values when each component was used in the past.

In other words, information on the flow of a people flow may be learned from the actual value when used in the past. Changing with a combination of components and a parameter as using, the information on the flow of a people flow may be stored along with the combination of the components and the parameter so that the info on the flow of a people flow can be predicted on the basis of the combination of components and the parameter when being used.

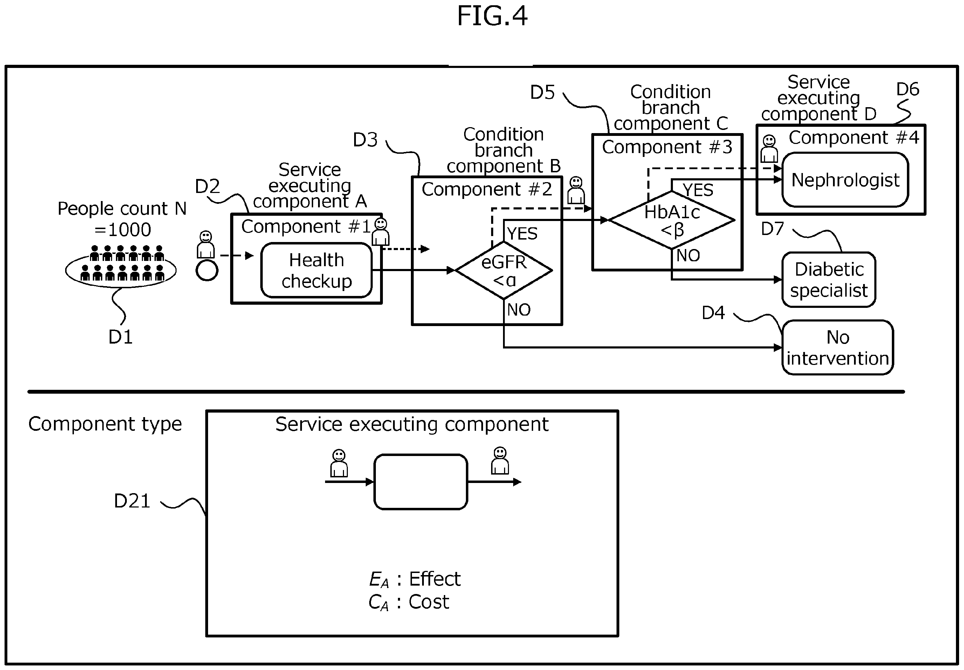

FIG. 4 is a diagram illustrating a service executing component included in the predicting model of the one embodiment.

The components indicated by the reference signs D1 to D7 in FIG. 4 are the same as the components indicated by the reference signs B1 to B7 in FIG. 2, respectively.

Influences (e.g., costs and effects) may be predicted by using a predicting model trained by accumulating information of influences (e.g., costs and effects) from actual values when each component was used in the past, the combination of components as using, and a parameter.

As illustrated in the reference sign D21, the effect E_{A} and the cost C_{A} when the service is executed on one person may be learned for each service executing component.

Since the effect E_{A} and the cost C_{A} vary with a parameter and a combination of components as using, the effect E_{A} and the cost C_{A} may be accumulated along with a parameter and a combination of components and can be predicted in combination with the parameter and the combination of components.

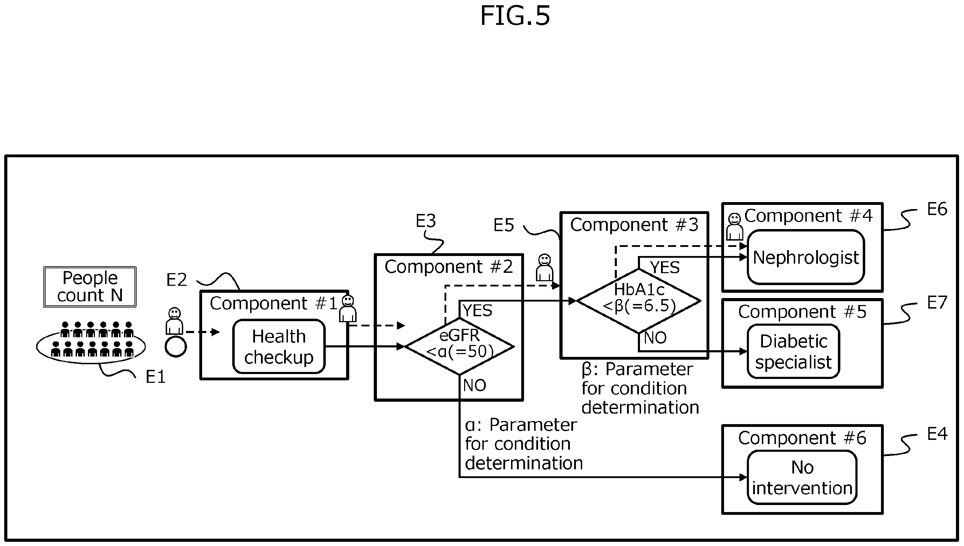

FIG. 5 is a diagram illustrating calculation of an effect and a cost of an overall policy in the predicting model of the one embodiment.

The effect and the cost per person are determined by using a regression model of the service executing components #4, #5, and #6.

In the example illustrated in FIG. 5, the people count N is inputted in the reference sign E1.

In the reference sign E2, the component #1 is set to "health checkup", and the sum of the people counts flowing into the component #1 and the effective are calculated. Assuming that the effect per person is E_{A}=f_{A_E} and the cost per person is C_{A}=f_{A_C}, when the people count is N, the sum of the effects is N × E_{A}, and the sum of costs is N× C_{A}.

In the reference sign E3, the component #2 is set to "eGFR<α(=50)", and the people count=N is inputted. The branch probability p_{B} that flows into Yes route of the reference sign E3 when the parameter α=50 is used in the order of the component #1 to the component #2 comes to be p_{B}=f_{B}(α=50) when the predicting model f_{B}(α) trained in the component #2 is used and the people count comes to be N×p_{B}.

If "eGFR<α" is not satisfied (see NO route of the reference sign E3), the component #6 is set to "no intervention" and it is determined as "no intervention" of the specialist is required for the citizen, as illustrated by the reference sign E4.

On the other hand, if "eGFR<α" is satisfied (see Yes route of the reference sign E3), the component #3 is set to "HbA1c<β(=6.5)" as illustrated in the reference sign E5. The branch probability p_{c} that flows into Yes route of the reference sign E5 when the parameters α=50 and β=6.5 are used in the order of the component #1, the component #2, and the component #3 comes to be p_{c}=f_{c}(α=50, β=6.5) when the predicting model f_{c}(α, β) trained in the component #2 and the people count is N×p_{B}×_{c}.

If "HbA1c<β" is satisfied (see YES route of the reference sign E5), as illustrated in the reference sign E6, the component #4 is set to "nephrologist", which means that intervention of a "nephrologist" is determined to be required for the citizen. In addition, the sum of the people counts flowing into the component #4, the sum of the effects, and the sum of the cost are calculated. Assuming that the effect per person is E_{A}=f_{E_E} and the cost per person is C_{A}=f_{E_C}, the people count is Nₚ=N×p_{A}×p_{B}, the sum of the effects is Nₚ×Eₚ, and the sum of the costs is Nₚ×C_{D}. For the components #5 and #6, the sum of the people counts and the sum of the effects are calculated likewise.

On the other hand, if "HbA1c<β" is not satisfied (see NO route of the reference sign E5), the component #5 is set to "diabetic specialist" and it is determined that intervention of a "diabetic specialist" is required for the citizen, as illustrated in the reference sign E7.

The effect of the entire policy is the total sum of the sum of the effects to people flowing into the component #1, the sum of the effects to people flowing into the component #4, the sum of the effects to people flowing into the component #5, and the sum of the effects to people flowing into the component #6. In addition, the cost of the entire policy is the total sum of the sum of the costs for people flowing into the component #1, the sum of the costs for people flowing into the component #4, the sum of the costs for people flowing into the component #5, and the sum of the costs for people flowing into the component #6.

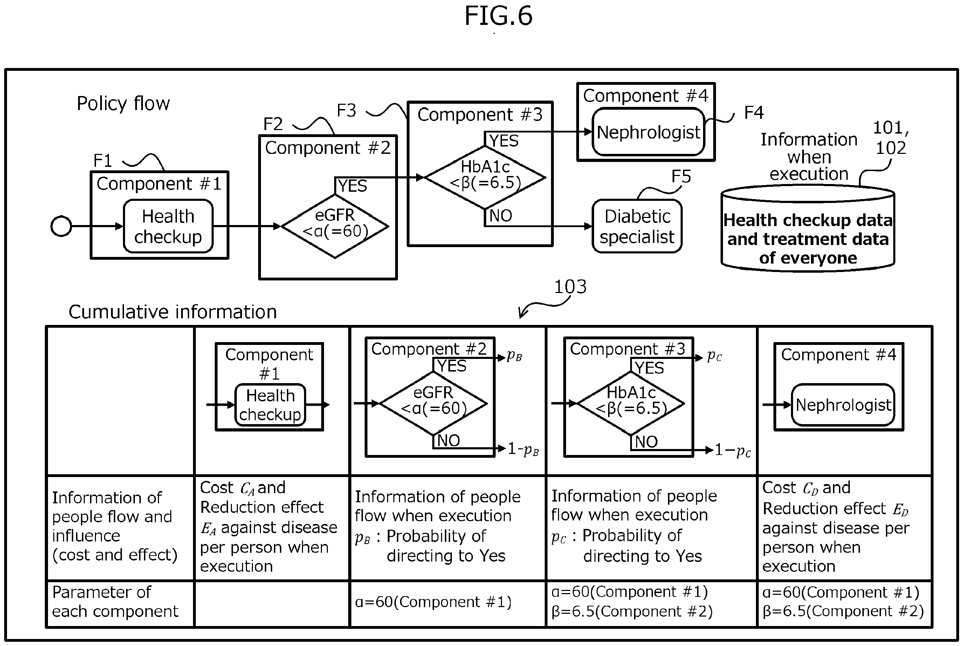

FIG. 6 is a diagram illustrating cumulative information 103 accumulated for training each component in the predicting model of the one embodiment.

The reference signs F1 to F5 illustrate components used in the past CKD follow-up policy executed in another City B.

In the reference sign F1, the component #1 is set to "health checkup".

In the reference sign F2, the component #2 is set to "eGFR<α(=60)".

If "eGFR<α(=60)" is satisfied (see YES route of the reference sign F2), the component #3 is set to "HbA1c<β(=6.5)" in the reference sign F3.

If "HbA1c<β(=6.5)" is satisfied (see Yes route of the reference sign F3), the component #4 is set to "nephrologist" in the reference sign F4, and it is determined that intervention of "Nephrologist" is required for the citizen.

On the other hand, if "HbA1c<β(=6.5)" is not satisfied (see NO route of the reference sign F4), it is determined that intervention of "diabetic specialist" is required for the citizen, as illustrated in the reference sign F5.

As information when execution of a policy flow, health checkup data 101 and treatment data 102 of the overall object persons are stored. Then, cumulative information 103 is generated from the data when the execution of the policy flow and accumulated.

In the cumulative information 103, information of people flow and an influence (i.e., costs and effects), and a parameter of each component are registered in association with one another for each of the components #1 to #4.

In the component #1, the cost C_{A} per person and the reduction effect E_{A} against disease per person when execution are registered as the information of people flow and an influence.

In the component #2, the probability pB of flowing into Yes route is registered as the information of people flow and an influence.

In the component #3, the probability pC of flowing into Yes route is registered as the information of people flow and an influence, and α=60 and β=6.5 are registered as parameters.

In the component #4, the cost C_{A} per person and the reduction effect E_{A} against disease per person when execution are registered as the information of people flow and an influence, and α=60 and β=6.5 are registered as parameters.

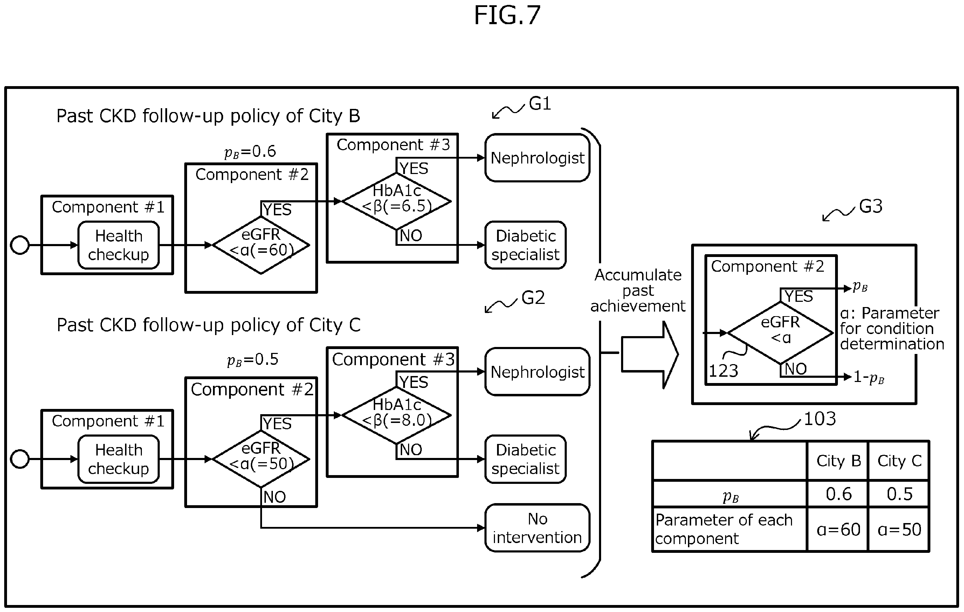

FIG. 7 is a diagram illustrating an accumulating process of the cumulative information 103 of the one embodiment.

The reference sign G1 illustrates the past CKD follow-up policy of City B, and the reference sign G2 illustrates the past CKD follow-up policy of City C.

Then, as illustrated in the reference sign G3, the past achievement in the component #2 is accumulated. For example, the probability p_{B} of flowing into the YES route and a parameter α for condition determination are accumulated.

In the example of FIG. 7, p_{B}=0.6 and α=60 are registered in relation to City B and p_{B}=0.5 and α=50 are registered in relation to City C in the cumulative information 103.

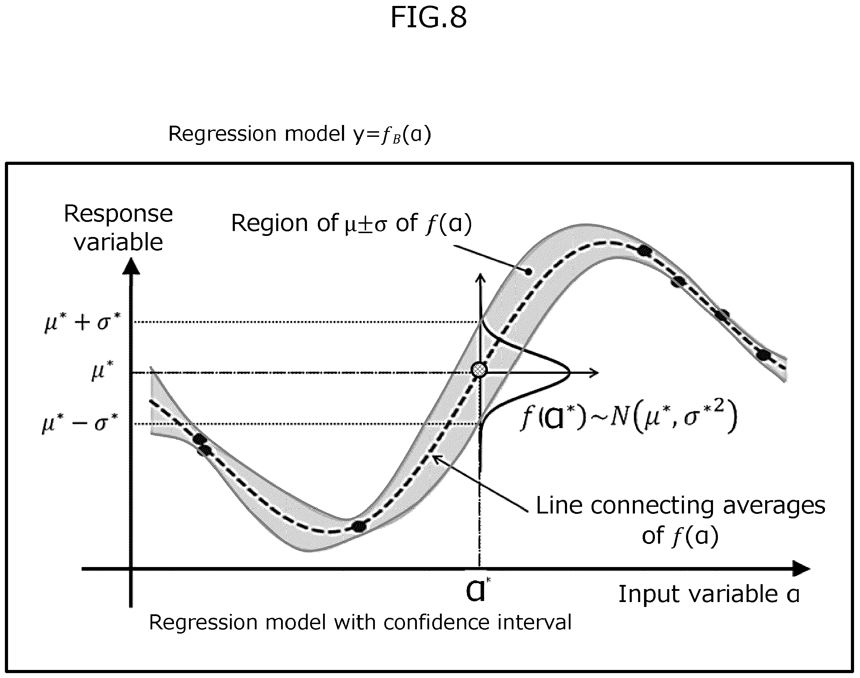

FIG. 8 is a diagram illustrating an example of a generating process of a regression model from a condition branch component included in the predicting model of the one embodiment.

A regression model f_{B}(α) of the branch probability p_{B} is trained with achievement when past execution. For example, the training may adopt one-dimensional Gaussian process regression. The input variable may be set to the parameter α of the component #2 illustrated in FIG. 7, and the response variable y may be set to the branch probability p_{B} of the component #2 illustrated in FIG. 7.

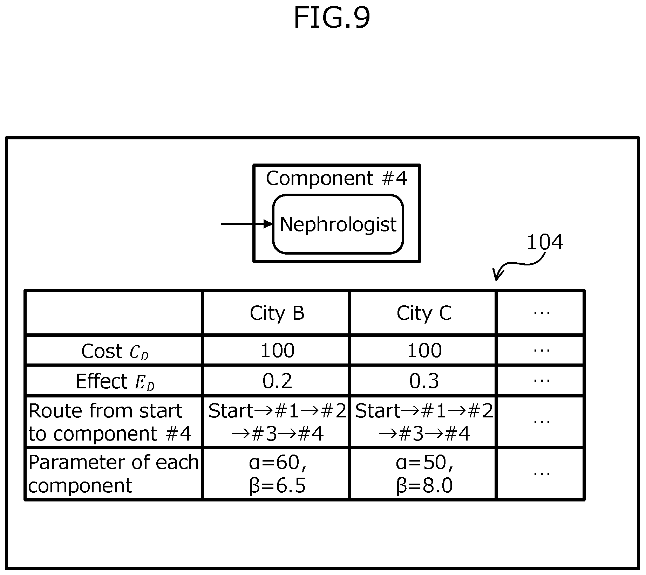

FIG. 9 is a diagram illustrating an example of a generating process of a regression model from a service executing component included in the predicting model of the one embodiment.

The summarized information 104 illustrated in FIG. 9 is generated as the data summarized in the component #4 illustrated in FIG. 7. In the example illustrated in FIG. 9, in relation to City B, the cost C_{D}=100, the effect E_{D}=0.2, the route from the start to the component #4 of "start → #1 → #2 → #3 → #4", the parameters α = 60 and β = 6.5 of each component are registered. In relation to City C, the cost C_{D}=100, the effect E_{D}=0.3, the route from start to the component #4 "start → #1 → #2 → #3 → #4", and the parameters α = 50 and β = 8.0 of each component are registered.

A regression model f_{D_E}(α, β) of the effect E_{D} and a regression model f_{D_C}(α, β) of the cost C_{D} are trained with achievement when being used in past.

For example, the training may adopt two-dimensional Gaussian process regression. The input variable may be set to the parameter α of the component #2 and also to the parameter β of the component #3 illustrated in FIG. 7, and the response variable may be set to the effect E_{D} and the cost C_{D}.

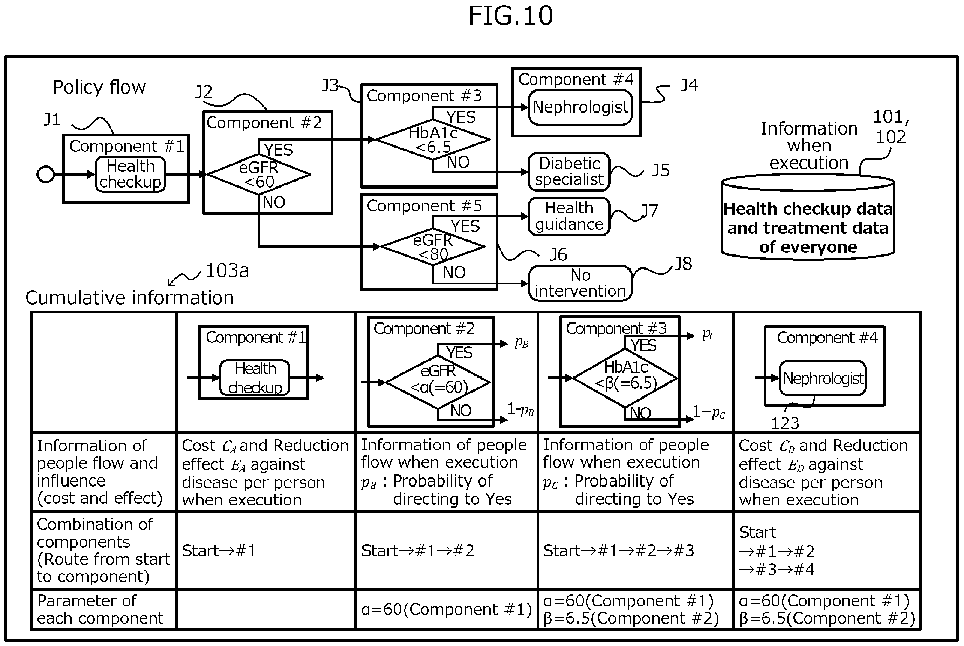

FIG. 10 is a diagram illustrating the cumulative information 103a accumulated for training each component by a predicting model according to a first modification.

The reference signs J1 to J8 illustrate the past CKD follow-up policy executed in another City B.

In the reference sign J1, the component #1 is set to "health checkup".

In the reference sign J2, the component #2 is set to "eGFR<α (=60)".

If "eGFR<α (=60)" is satisfied (see YES route of the reference sign J2), the component #3 is set to "HbA1c<β (=6.5)" in the reference sign J3.

If "HbA1c<β (=6.5)" is satisfied (see Yes route of the reference sign J3), the component #4 is set to "nephrologist" in the reference sign J4, and it is determined that intervention of "Nephrologist" is required for the citizen.

On the other hand, when "HbA1c<β (=6.5)" is not satisfied (see NO route of the reference sign J4), it is determined that intervention of "diabetic specialist" is required for the citizen, as illustrated in the reference sign J5.

If "eGFR<α (=60)" is not satisfied in the reference sign J2 (see NO route of the reference sign J2), the component #5 is set to "eGFR<α(=80)" in the reference sign J6.

If "eGFR<α(=80)" is satisfied (see YES route of the reference sign J6), it is determined that "health guidance" to the citizen is required in the reference sign J6.

On the other hand, if "eGFR<α(=80) " is not satisfied (see NO route of the reference sign J6), it is determined as "No intervention" is required for the citizen as illustrated in the reference sign J8.

As information when execution of a policy flow, all health checkup data 101 and treatment data 102 of the overall object persons are stored. Then, cumulative information 103a is generated from the data when the execution of the policy flow and accumulated.

In the cumulative information 103a, information of people flow and an influence (i.e., costs and effects), a combination of components (i.e., route from the start to the component), and a parameter of each component are registered in association with one another for each of the components #1 to #4.

In the component #1, the cost C_{A} per person and the reduction effect E_{A} against disease per person when execution are registered as the information of people flow and an influence, and a combination "start → #1" of components is registered.

In the component #2, the probability pE of flowing into Yes route is registered as the information of people flow and an influence, and a combination "start → #1→#2" of components is registered.

In the component #3, the probability pC of flowing into Yes route is registered as the information of people flow and an influence, α=60 and β=6.5 are registered as parameters, and the combination "start→#1→#2→#3" of components is registered.

In the component #4, the cost C_{A} per person and the reduction effect E_{A} against disease per person when execution are registered as the information of people flow and an influence, α=60 and β=6.5 are registered as parameters and the combination "Start→#1→#2→#3→#4" of components is registered.

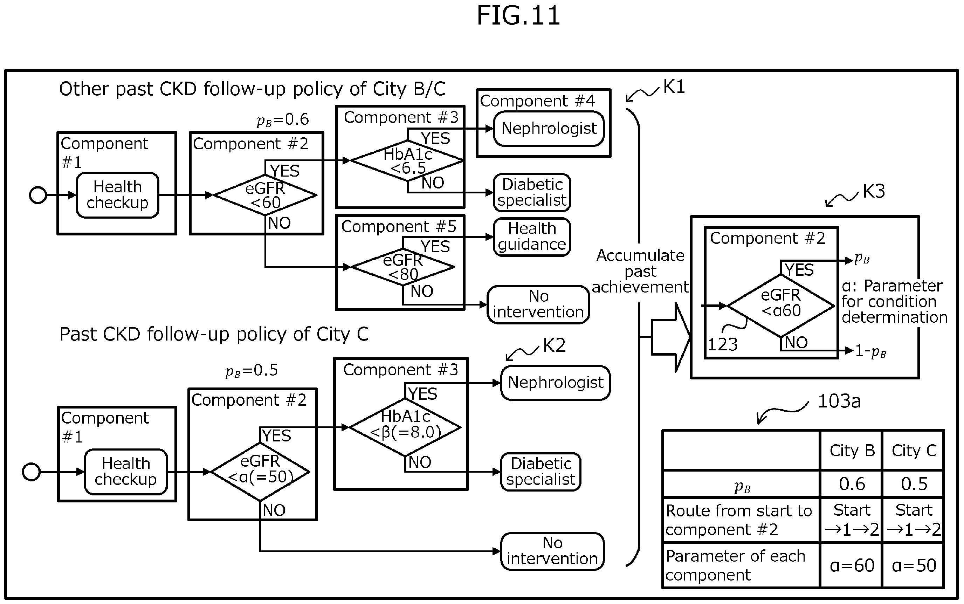

FIG. 11 is a diagram illustrating an accumulating process of the cumulative information 103a of the first modification.

The reference sign K1 illustrates the past CKD follow-up policy of City B, and the reference sign K2 illustrates the past CKD follow-up policy of City C.

Then, as illustrated in the reference sign K3, the past achievement in the component #2 is accumulated. For example, the probability p_{B} of flowing into Yes route, a route from the start to the component #2 (i.e., the route information) and a parameter α for condition determination are accumulated.

In the example of FIG. 11, p_{B}=0.6, the route "start → #1 → #2", and α=60 are registered in relation to City B, and p_{B}=0.5, the route "start → #1 → #2", and α=50 are registered in relation to City C in the cumulative information 103.

A regression model f_{B} (α) (see FIG. 8) of the branch probability p_{B} is trained with achievement when the route "start→#1→#2" was previously used in this order. For example, the training may adopt one-dimensional Gaussian process regression. The input variable may be set to the parameter α of the component #2 illustrated in FIG. 11, and the response variable may be set to the branch probability p_{B} of the component #2 illustrated in FIG. 11.

A regression model f_{D_E}(α, β) of the effect E_{P} and a regression model f_{D_C}(α, β) of the cost C_{D} are trained from achievement when the route "start→#1→#2→#3→#4" was previously used in this order (see summarized information 104 in FIG. 9). For example, the training may adopt two-dimensional Gaussian process regression. The input variable may be set to the parameter α of the component #2 and also to the parameter β of the component #3 illustrated in FIG. 11, and the response variable may be set to the effect E_{D} and the cost C_{D}.

By using the regression model illustrated in FIG. 8, the values of the branch probability and the effect can be obtained values with confidence intervals. A confidence interval is an interval represented by µ^{∗}-σ^{∗}<γ<µ^{∗}+σ^{∗} interval. The cost and the effect of the entire policy may be predicted to be values with confidential intervals by using information of the confidential intervals of the branch probability and the effect of each component.

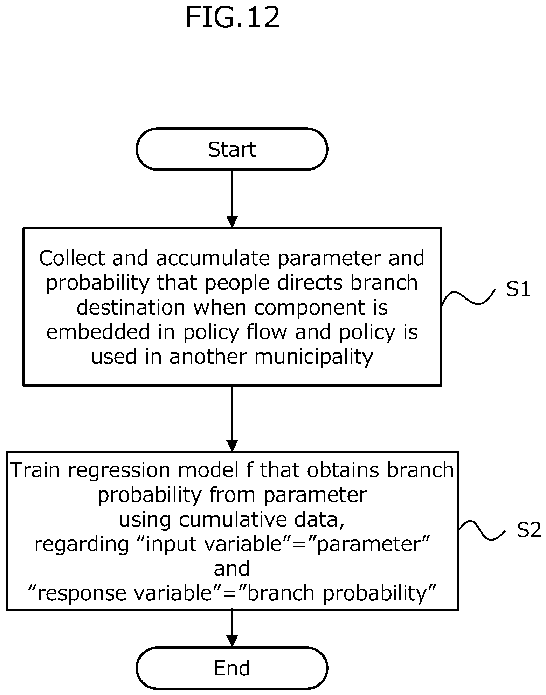

Description will now be made in relation to a constructing process of a predicting model of the one embodiment with reference to the flow diagram (Steps S1 and S2) of FIG. 12.

A parameter and a probability that people directs a branch destination when the component is embedded in a policy flow and policy is used in another municipality are collected and accumulated (Step S1).

The parameter and the branch probability are respectively regarded as the input variable and the response variable on the basis of the accumulated data, the branch probability is obtained from the parameter, and the regression model f is trained (Step S2).

Then, the constructing process of a predicting model ends.

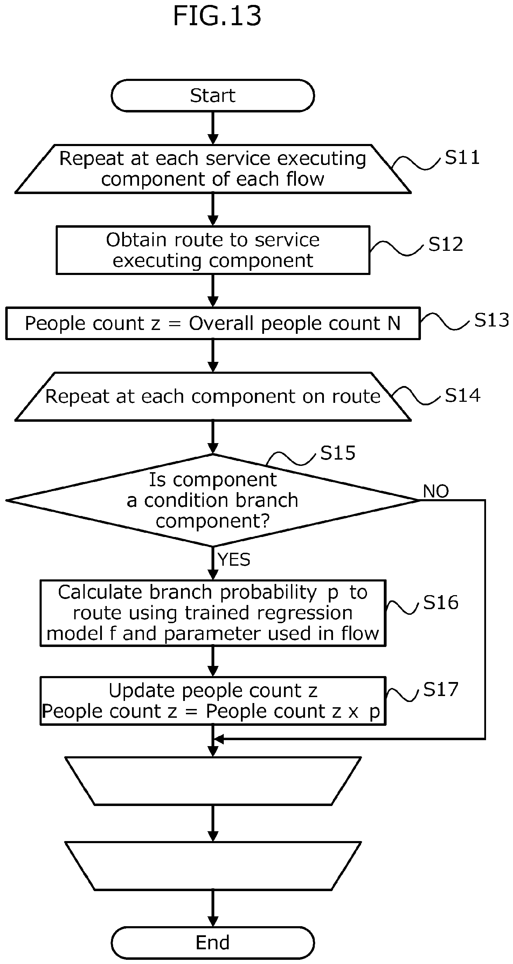

Next, description will now be made in relation to a calculating process of a probability, using a predicting model, in the one embodiment with reference to the flow diagram (Steps S11 to S17) of FIG. 13.

The following process of Steps S12 to S17 is repeatedly executed on each service executing component of each flow (Step S11).

The route to the service executing component is obtained (Step S12).

The people count z = the overall people count N is set (Step S13).

The following Steps S15 to S17 are repeatedly executed on each component on the route (Step S14).

A determination is made as to whether the component is a condition branch component (Step S15).

If the component is not a condition branch component (see NO route in step S15), the repeat of Steps S15 to S17 (see Step S14) are skipped and a next loop of the process is executed on another component.

On the other hand, if the component is a condition branch component (see YES route in step S15), the branch probability p to the route is calculated by using the trained regression model f and the parameter used in the flow (Step S16).

The people count z is updated and set to the people count z = people count z × **p** (Step S17). Then, when the repetition of Steps S15 to S17 (see Step S14) and the repetition of the Step S12 to 17 (see Step S11) are completed, the calculating process of a probability using the predicted model ends.

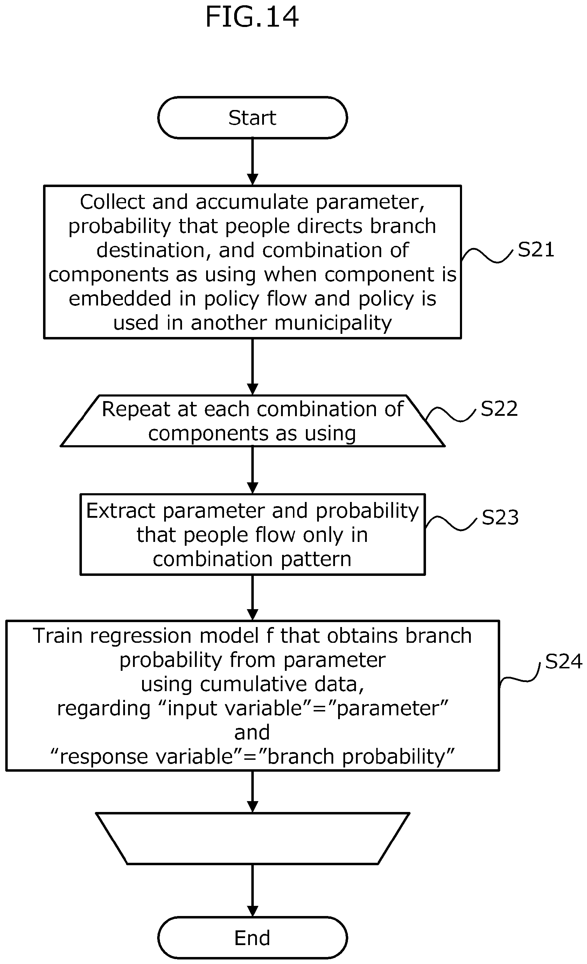

Next, description will now be made in relation to a constructing process of a predicting mode of a first example according to t first modification with reference to the flow diagram (Steps S21 to S24) of FIG. 14.

A parameter and a probability that people directs a branch destination combination of components as using when the component is embedded in policy flow and policy is used in another municipality are collected and accumulated (Step S21) .

The following Steps S23 and S24 are repeatedly performed on each combination of components as using (Step S22).

A parameter and a probability that people flow only in relation to a pattern of the combination are extracted (Step S23) .

The parameter and the branch probability are respectively regarded as the input variable and the response variable on the basis of the accumulated data, the branch probability is obtained from the parameter, and the regression model f is trained (Step S24). Then, when the repeat of Steps S23 and S24 (see Step S22) is completed, the first example in the constructing process of a predicting model according to the first modification ends.

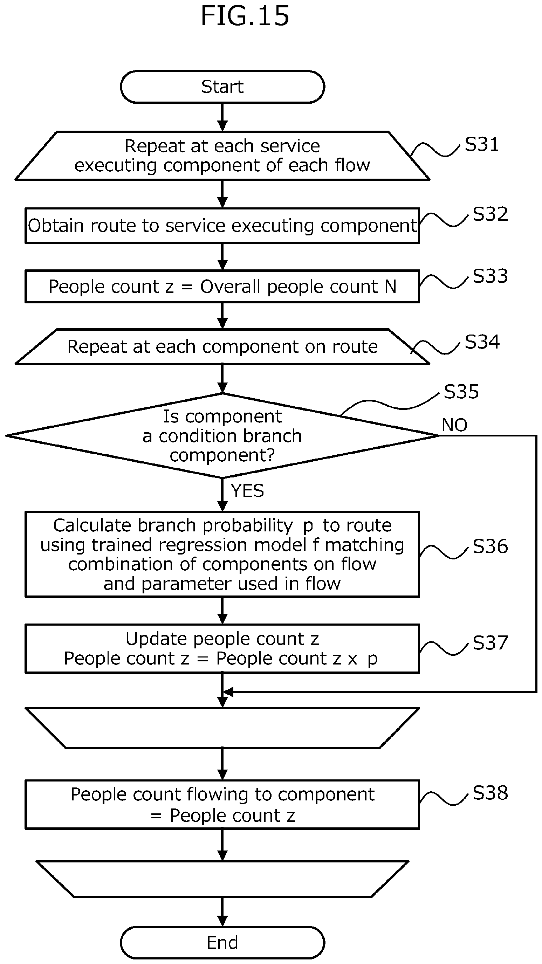

Next, description will now be made in relation to a first example of a calculating process of a probability using a predicting model according to the first modification with reference to a flow diagram (Steps S31 to S38) of FIG. 15.

The following process of Steps S32 to S38 is repeatedly executed on each service executing component of each flow (Step S31).

The route to the service executing component is obtained (Step S32).

The people count z = the overall people count N is set (Step S33).

The following Steps S35 to S37 are repeatedly executed on each component on the route (Step S34).

A determination is made as to whether the component is a condition branch component (Step S35).

If the component is not a condition branch component (see NO route in Step S35), when the repeat of Steps S55 to S37 (see Step S34) is completed, the process proceeds to Step S38.

On the other hand, if the component is a condition branch component (see YES route in Step S35), the branch probability p to the route is calculated by using the trained regression model f coinciding with the combination of components of the flow and the parameter used in the flow (Step S36) .

The people count z is updated and set to the people count z = people count z × p (Step S37). Then, when the repeat of Steps S35 to S37 (see Step S34) is completed, the process proceeds to Step S38.

The number of people flowing into the component = people count z is set (Step S38). Then, when the repeat of the Steps S32 to S38 (see Step S31) is completed, the first example of the process of calculating a probability using a predicting model of the first modification ends.

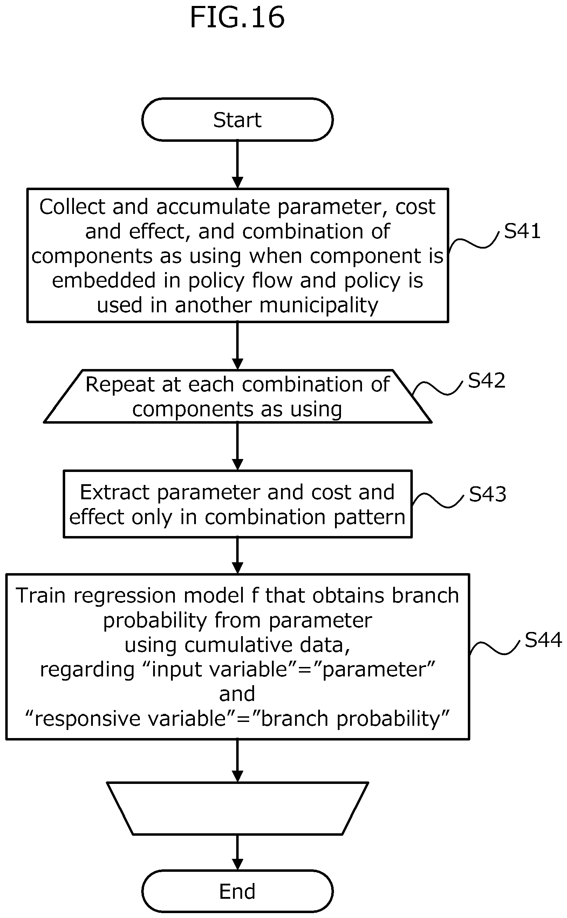

Next, description will now be made in relation to a constructing process of a predicting mode of a second example according to the first modification with reference to the flow diagram (Steps S41 to S44) of FIG. 16.

A parameter, the cost and the effect, and a combination of a component as using when the component is embedded in policy flow and policy is used in another municipality are collected and accumulated (Step S41).

The following Steps S43 and S44 are repeatedly performed on each combination of components as using (Step S42).

A parameter and a cost and an effect only in relation to a pattern of the combination are extracted (Step S43).

The parameter and the cost and the effect are respectively regarded as the input variable and the response variable on the basis of the accumulated data, the cost and the effect are obtained from the parameter, and the regression model f is trained (Step S44).

Then, when the repetition of Steps S43 and S44 (see Step S42) is completed, the second example in the constructing process of a predicting model according to the first modification ends.

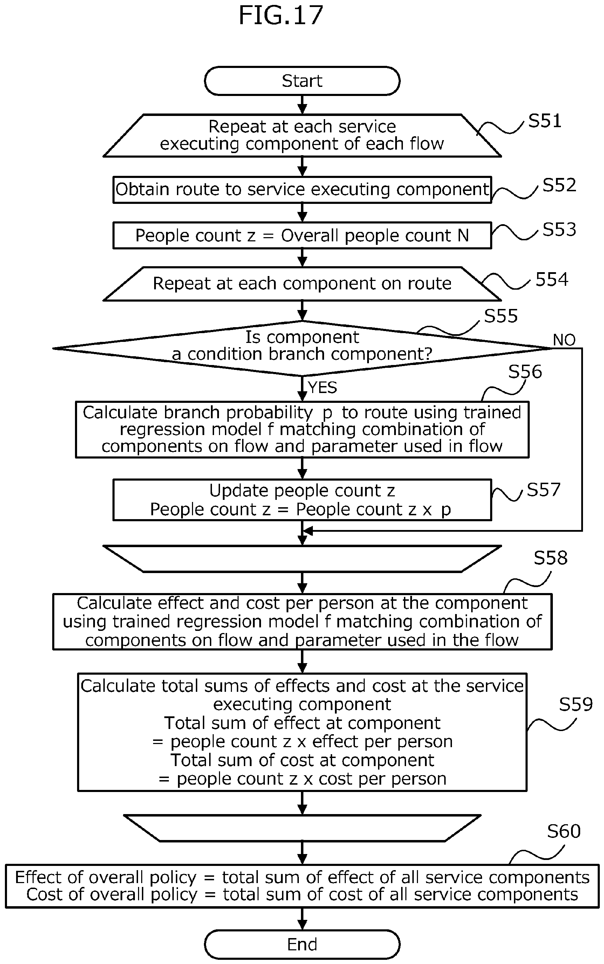

Next, description will now be made in relation to a second example of a calculating process of a probability using a predicting model according to the first modification with reference to a flow diagram (Steps S51 to S60) of FIG. 17.

The following process of Steps S52 to S59 is repeatedly executed on each service executing component of each flow (Step S51).

The route to the service executing component is obtained (Step S52).

The people count z = the overall people count N is set (Step S53).

The following Steps S55 to S57 are repeatedly executed on each component on the route (Step S54).

A determination is made as to whether the component is a condition branch component (Step S55).

If the component is not a condition branch component (see NO route in Step S55), when the repeat of Steps S55 to S57 (see Step S54) is completed, the process proceeds to Step S58.

On the other hand, if the component is a condition branch component (see YES route in Step S55), the branch probability p to the route is calculated by using the trained regression model f coinciding with the combination of components of the flow and the parameter used in the flow (Step S56) .

The people count z is updated and set to the people count z = people count z × p (Step S57). Then, when the repeat of Steps S55 to S57 (see Step S54) is completed, the process proceeds to Step S58.

The cost and the effect per person at the component are calculated by using the trained regression model f coinciding with the combination of components of the flow and the parameter used in the flow (Step S58).

The total sum of effects and the costs at the service executing component are calculated (Step S59). The total sum of the effects at a component = the people count z × the effect per person, and the total sum of the costs at a component = the number of people z × the cost per person. Then, when the repeat of Steps S52 to S57 (see Step S51) is completed, the process proceeds to Step S60.

The effect of the entire policy is set to the total sum of the effects of all the service executing components and the cost of the entire policy is set to the total sum of the costs of all the service executing components (Step S60). Then, the second example of the calculating process of a probability using a predicting model of the first modification ends.

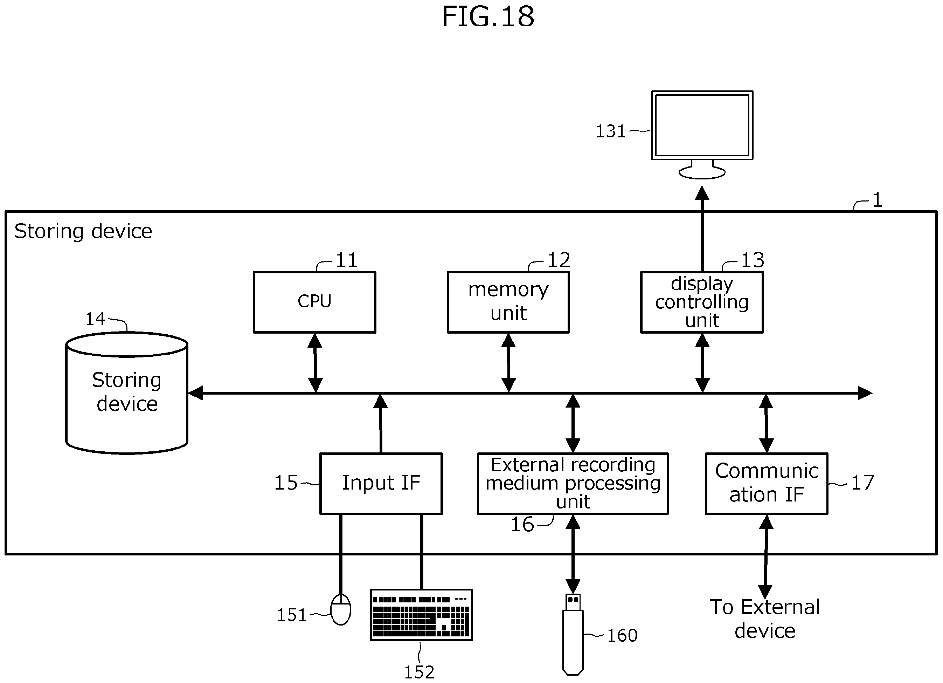

FIG. 18 is a diagram schematically illustrating an example of a hardware configuration of an information processing apparatus of the one embodiment and the modifications.

As illustrated in FIG. 18, the information processing apparatus 1 includes a CPU 11, a memory unit 12, a display controlling unit 13, a storing device 14, an input IF 15, an external recording medium processing unit 16, and a communication IF 17.

The memory unit 12 is an example of a storing device, and is exemplified by a Read Only Memory (ROM) and a Random Access Memory (RAM). In the ROM of the memory unit 12, a program such as Basic Input/Output System (BIOS) may be written. The software program of the memory unit 12 may be appropriately read and executed by the CPU 11. The R_AM of the memory unit 12 may be used as a temporary recording memory or a working memory.

The display controlling unit 13 is connected to a displaying device 131, and controls the displaying device 131. Examples of the displaying device 131 are a liquid crystal display, a Light-Emitting Diode (OLED) display, a Cathode Ray Tube (CRT), an electronic paper display, which present various types of information to the operator or the like. The displaying device 131 may be combined with an input device and is exemplified by a touch panel.

The storing device 14 is a high IO performance storing device and is exemplified by a Dynamic Random Access Memory (DRAM), an SSD, a Storage Class Memory (SCM), and a HDD.

The input IF 15 is connected to an input device such as a mouse 151 and a keyboard 152 and controls the input device such as the mouse 151 and the keyboard 152. The mouse 151 and the keyboard 152 are examples of an input device through which the operator makes various inputting operations.

The external recording medium processing unit 16 is configured to be capable of mounting a recording medium 160 thereon. The external recording medium processing unit 16 is configured to be capable of reading, in a state where the recording medium 160 is mounted on the external recording medium processing unit 16, the information recorded in the recording medium 160. In the present embodiment, the recording medium 160 is portable. Examples of the recording medium 160 is a flexible disc, an optical disc, a magnetic disc, a magneto-optical disc, and a semiconductor memory.

The communication IF 17 is an interface that makes the information processing apparatus 1 communicable with an external apparatus.

The CPU 11 is an example of a processor (in other words, a computer), and a processor device that carries out various controls and arithmetic operations. The CPU 11 achieves various functions by executing an Operating System (OS) and a program read in the memory unit 12. The CPU 11 may be a multiprocessor including multiple CPUs, a multi-core processor including multiple CPU cores, or a configuration including multiple multi-core processors.

The apparatus that controls the entire operation of the information processing apparatus 1 is not limited to the CPU 11, and may alternatively be any one of MPUs (Micro Processing Units), DSPs (Digital Signal Processors), ASICs (Application Specific Integrated Circuits), PLDs (Programmable Logic Devices), FPGAs (Field Programmable Gate Arrays), and may be a combination of two or more of the above.

### (C) Effect:

According to a method for generating a predicting model, an information processing apparatus, and a program for generating a predicting model according to the above embodiment can bring the following effects and advantages, for example.

The CPU 11 accumulates multiple combinations of a parameter representing a branch condition and a branch probability in relation to one or more condition branch components in a workflow. The CPU 11 generates, using the multiple accumulated combinations, a model that predicts a branch probability corresponding to a parameter used when a particular condition branch component among the one or more condition branch components is used.

This can generate a highly accurate predicting model with low computational complexity. Specifically, since this eliminates the requirements for prediction for each individual and consequently hundreds of thousands of computations, the people count directing each service can be predicted with low computational complexity, which is several multiple of the number of components.

The CPU 11 calculates, when the particular condition branch component receives an input of a first parameter, a first branch probability representing an outputted value satisfying the received first parameter.

This can efficiently predict the accurate people count directing each service.

In the accumulating of the multiple combinations, the CPU 11 accumulates the multiple combinations including the branch probability, the parameter, and route information representing a route passing through the multiple condition branch components in association with one another. In the calculating of the first branch probability, the CPU 11 calculates the first probability when the route to the particular condition branch component satisfies the route information.

With this configuration, if distribution of people on the basis of a condition branch component in a flow is affected by influence of another condition branch component at the upstream side of the flow, the distribution can be predicted, considering the influence.

In the accumulating of the multiple combinations, the CPU 11 accumulates, in association with one another, the plurality of combinations including the branch probability, the parameter, and route information representing a route passing through the one or more condition branch components, an influence value when the one or more condition branch components included in the route information and one or more service executing component are used In the generating of the model, the CPU 11 generates, when a route to the particular condition branch component satisfies the route information, a model that predicts the branch probability and an influence value when the particular condition branch component is used.

Specifically, since this eliminates the requirements for prediction for each individual and consequently influence (e.g., cost and effect) of the policy can be predicted with low computational complexity, which is several multiple of the number of components.

### (D) Miscellaneous:

The disclosed techniques are not limited to the embodiment described above, and may be variously modified without departing from the scope of the present embodiment. The respective configurations and processes of the present embodiment can be selected, omitted, and combined according to the requirement.

The above embodiment generates a predicting model for a workflow in a governmental health service, but is not limited to this. The above embodiment may alternatively be used to generate a predicting model for various workflows, such as operations, tests, and questionnaire with condition branches. These alternatives can obtain the same effects and advantages as those of the above embodiment.

### [Description of Reference Sign]

- 1:: information processing apparatus
- 11:: CPU
- 12:: memory unit
- 13:: display controlling unit
- 14:: storing device
- 16:: external recording medium processing unit
- 101:: health checkup data
- 102:: treatment data
- 103,103a:: cumulative information
- 103a:: cumulative information
- 104:: summarized information
- 131:: displaying device
- 151:: mouse
- 152:: keyboard
- 160:: recording medium
- 15:: input interface
- 17:: communication

## Claims

1. A computer-implemented method for generating a predicting model comprising:
accumulating a plurality of combinations of a parameter representing a branch condition and a branch probability in relation to one or more condition branch components in a workflow; and
generating, using the plurality of combinations accumulated, a model that predicts a branch probability corresponding to a parameter used when a particular condition branch component among the one or more condition branch components is used.

2. The computer-implemented method according to claim 1, further comprising
when the particular condition branch component receives an input of a first parameter, calculating a first branch probability representing an outputted value satisfying the received first parameter.

3. The computer-implemented method according to claim 2, wherein:
the accumulating of the plurality of combinations accumulates the plurality combinations including the branch probability, the parameter, and route information representing a route passing through a plurality of the condition branch components in association with one another; and
the calculating of the first branch probability calculates the first probability when the route to the particular condition branch component satisfies the route information.

4. The computer-implemented method according to any one of claims 1-3, wherein:
the accumulating of the plurality of combinations accumulates, in association with one another, the plurality of combinations including the branch probability, the parameter, and route information representing a route passing through the one or more condition branch components, an influence value when the one or more condition branch components included in the route information and one or more service executing component are used; and
the generating of the model generates, when a route to the particular condition branch component satisfies the route information, a model that predicts the branch probability and an influence value when the particular condition branch component is used.

5. An information processing apparatus comprising:
a processor being configured to:
accumulate a plurality of combinations of a parameter representing a branch condition and a branch probability in relation to one or more condition branch components in a workflow; and
generate, using the plurality of combinations accumulated, a model that predicts a branch probability corresponding to a parameter used when a particular condition branch component among the one or more condition branch components is used.

6. The information processing apparatus according to claim 5, wherein the processor is further configured to
when the particular condition branch component receives an input of a first parameter, calculate a first branch probability representing an outputted value satisfying the received first parameter.

7. The information processing apparatus according to claim 6, wherein the processor is further configured to:
in the accumulating of the plurality of combinations, accumulate the plurality combination including the branch probability, the parameter, and route information representing a route passing through a plurality of the condition branch components in association with one another; and
in the calculating of the first branch probability, calculate the first probability when the route to the particular condition branch component satisfies the route information.

8. The information processing apparatus according to any one of claims 5-7, wherein the processor is further configured to:
in the accumulating of the plurality of combinations, accumulate, in association with one another, the plurality of combinations including the branch probability, the parameter, and route information representing a route passing through the one or more condition branch components, an influence value when the one or more condition branch components included in the route information and one or more service executing component are used; and
in the generating of the model, generate, when a route to the particular condition branch component satisfies the route information, a model that predicts the branch probability and an influence value when the particular condition branch component is used.

9. A predicting model generating program for causing a computer to execute a process comprising:
accumulating a plurality of combinations of a parameter representing a branch condition and a branch probability in relation to one or more condition branch components in a workflow; and
generating, using the plurality of combinations accumulated, a model that predicts a branch probability corresponding to a parameter used when a particular condition branch component among the one or more condition branch components is used.

10. The predicting model generating program according to claim 9, wherein the process further comprises
when the particular condition branch component receives an input of a first parameter, calculating a first branch probability representing an outputted value satisfying the received first parameter.

11. The predicting model generating program according to claim 10, wherein the process further comprises:
in the accumulating of the plurality of combinations, accumulating the plurality combination including the branch probability, the parameter, and route information representing a route passing through a plurality of the condition branch components in association with one another; and
in the calculating of the first branch probability, calculating the first probability when the route to the particular condition branch component satisfies the route information.

12. The predicting model generating program according to any one of claims 9-11, wherein the process further comprises:
in the accumulating of the plurality of combinations, accumulating, in association with one another, the plurality of combinations including the branch probability, the parameter, and route information representing a route passing through the one or more condition branch components, an influence value when the one or more condition branch components included in the route information and one or more service executing component are used; and
in the generating of the model, generating, when a route to the particular condition branch component satisfies the route information, a model that predicts the branch probability and an influence value when the particular condition branch component is used.
